# EUROPEAN PATENT SPECIFICATION

(11) **EP 0 454 315 B1**
(45) Date of publication and mention of the grant of the patent: **14.06.1995**
(21) Application number: 91303083.9
(22) Date of filing: 08.04.1991
(51) Int. Cl.: B01L 3/00, G01N 33/53

(54) **An assay tray assembly**
Analyseplatte
Plateau d'analyse

(30) Priority: 25.04.1990 GB 9009308
(43) Date of publication of application: 30.10.1991
(73) Proprietor: Pfizer Limited, Sandwich Kent CT13 9NJ (GB); PFIZER INC., New York, N.Y. 10017 (US)
(72) Inventor: Merson, James Richard, Dr., Ash Nr. Canterbury, Kent, CT3 2AB (GB); Bojanic, Dejan, Dr., Kent, CT 11 7BW (GB)
(74) Representative: Walters, Frederick James

(56) References cited:
- EP-A- 0 339 769
- EP-A- 0 359 249
- WO-A-85/03886
- US-A- 4 111 754
- US-A- 4 599 315

## Description

The present invention relates to an assay tray assembly. It is particularly concerned with an assembly for use in the testing or analysis of liquids, usually of a chemical, bio-chemical or biological nature, by a batch process whereby multiple small quantities of the liquid or liquids under test can be prepared for analysis substantially simultaneously, the process conveniently being carried out by conventional mechanical handling equipment. Assemblies of the kind mentioned include an separation medium to which the liquid for analysis is subjected; this medium may simply serve to remove solid/particulate matter from the liquid by filtration or may be in the form of chromatographic medium having a selectively adsorbent nature intended to separate or indicate a particular characteristic (or the lack of such a characteristic) of the liquid under test.

Particularly in chemical and pharmaceutical research there is a large demand for the assay or analysis of small quantities of liquid by chromatographic techniques and it has hitherto been proposed to provide assay trays and assemblies whereby individual samples of test liquid are prepared for and subjected to analysis by multi-batch processing on a microtiter principle, for example as disclosed in EP-A-0087899 in which samples of a reaction medium are carried by a spaced array of downwardly extending rods which rods and samples are inserted, one each, into a corresponding array of discrete wells containing the test liquid. An assay tray assembly for similar batch processing is also disclosed in U.K. Patent Specification GB-A-2,188,418 in which a base tray has a plurality of reaction wells and an overlying tray has a corresponding number of solid or hollow reaction projections either or both of which may carry a reaction medium that provides a basis for analysis when the reaction projections are inserted into the corresponding reaction wells containing the test liquid.

A conventional assay technique is to locate the separation medium in an upstanding tubular chamber having an open top and a bottom outlet and to pipette or otherwise deposit the liquid under test into the chamber to flow through or over the extraction medium prior to emerging from the bottom outlet. Conventionally this type of analysis is made on an individual basis, for example by use of an extraction cartridge sold under the Trade Mark BOND ELUT which comprises a tubular plastics body carried within which is a predetermined chromatographic medium. Such individual cartridges have been found inappropriate in trying to achieve a high through put of test samples for analysis. It has however been proposed, in U.S. Patent US-A-4,090,850 to provide an assay unit (particularly for radio-immunio assay) which utilises a batch process whereby a tray is provided with multiple wells each having at its bottom an outlet communicating with a vacuum chamber. Each well carries an extraction medium so that liquid under test deposited in each well flows through the extraction medium and the percolate or eluate, as the case may be, passes by way of the outlets into the vacuum chamber which acts as a common waste reservoir. By exhausting the vacuum chamber the liquids in the wells are subjected to a pressure differential to increase the rate of through-flow for the analysis procedure.

A further assay tray assembly for batch processing is disclosed in EP-A-0 359 249 which is believed to be the most relevant prior art to the present invention and provides the basis for the introductory part that precedes the characterizing clause of claim 1 hereof.

With assay techniques in which there is a flow of liquid under test through or over a separation medium as previously discussed, it is frequently important that the liquid emerging from the separation medium is available for discrete analysis, possibly as an alternative, or addition, to the analysis of the separation medium. If such a facility is to be provided in the assaying of liquids by use of a batch technique, it is essential that there is no intermixture or cross contamination between the liquids emerging from the individual outlets of the several chambers which accommodate the separation medium. It is an object of the present invention to provide an assay tray assembly by which this requirement may be satisfied and which may be produced relatively inexpensively for one-time or repeated use, is convenient to use, and lends itself to mechanical handling and processing, for example with apparatus having an automatic bulk dispensing and aspirating capability.

### STATEMENTS OF INVENTION & ADVANTAGES

According to the present invention there is provided an assay tray assembly comprising a plastics moulded test tray removably mounted in overlying relationship in engagement with a plastics moulded collection tray; the test tray having a spaced array of discrete identical upstanding chambers to accommodate a predetermined volume of liquid for analysis, each chamber being formed with a top opening and a bottom opening for the flow therethrough of the liquid, said bottom opening being located in a downwardly projecting tubular spigot for the respective chamber and each said chamber carrying a separation medium to which the liquid is to be subjected during its said through-flow; the collection tray having discrete upstanding wells each with an upwardly opening mouth, said wells corresponding in number to the chambers and having their mouths disposed in a spaced array corresponding to that of the tubular spigots; the tubular spigots being received one in each of said well mouths for the respective wells to collect liquid emanating from the chambers and to alleviate cross contamination between such liquids and abutment means being provided between the trays to restrict the depth of entry of the tubular spigots into the wells CHARACTERISED IN THAT the test tray is formed as a one piece plastics moulding and each chamber of the test tray has a cylindrical lower part length which communicates with the bottom opening, said bottom opening being formed as an outlet port by an inwardly directed annular flange provided in the tubular spigot part of the respective chamber; the cylindrical lower part length of each chamber carrying said separation medium with the medium in cylindrical face-to-face contact with the cylindrical lower part length of the chamber in which it is carried and said medium being retained in the respective chamber by abutment with the flange which forms the outlet port of that chamber, and wherein the volume of each well below the tubular spigot it receives is greater than the volume of a liquid which it is intended to receive from the chamber communicating therewith.

By the present invention the tubular spigots of the chambers are received one each in the mouths of the discrete wells in the collection tray. This ensures that the liquid (or liquids) which is under test will flow over or through the separation medium and by way of the opening in the bottom of its respective chamber into a particular well of the collection chamber without unintentional seepage or cross contamination of such liquid between the adjacent chambers or wells. Furthermore, in the assembly of the invention it is intended that the volume of each well is capable of accommodating below the tubular spigot with which it communicates, all of the liquid (the percolate or eluate) which passes through the tubular spigot and is derived from the liquid initially deposited in the corresponding chamber. It is envisaged that the liquid under test will usually be pipetted into the chambers in predetermined volumes, possibly to the extent that a particular chamber is full of such liquid above the separation medium and the volume of the well should be capable of accommodating an equivalent volume of liquid below and out of contact with the tubular spigot. By ensuring that when the test and collection trays are fitted together with the tubular spigots engaging in the respective wells and each well has adequate capacity to accommodate, beneath the tubular spigot, the whole of the liquid through flow, it will be appreciated that such liquid is remote from the separation medium and the tubular spigots and the possibility is alleviated of liquid seeping from one well to another and possibly dripping from the spigots to cause cross contamination when the two trays are separated. The samples of liquid in the wells can therefore confidently be subjected to independent analysis.

The trays are preferably formed as one piece injection mouldings with their respective chambers and wells disposed in a conventional microtiter plate layout, that is arrayed in columns and rows extending perpendicularly thereto. A typical such array has twelve parallel columns and eight parallel rows to provide ninety six chambers or wells in the respective trays (this latter array is likely to prove most acceptable for automatic handling of the trays by multiple batch pipetting equipment which is currently available). The trays will usually have moulded-in indicia for individually identifying each chamber or well as the case may be and similar indicia will normally be provided for both the test tray and the collection tray. Orientating means may be provided which permits engagement between the two trays only when they are mounted in their correct overlying relationship in which it is ensured that the two trays are correctly orientated with respect to each other (that is when the individually identified tubular spigots engage within correspondingly individually identified wells). The orientating means can conveniently comprise moulded-in tracks, projections, recesses or rebates on the trays which are intended to inter-engage between the trays when the trays are correctly mounted (or possibly to engage with mechnical handling equipment to ensure that the test and collection trays are correctly orientated with respect to each other and in the equipment) prior to the chambers being charged with the liquid under test.

The chambers are preferably formed by downwardly extending tubes in a substantially parallel array which tubes may be maintained in their spaced array by upstanding webs of the test tray that interconnect each such tube to the tubes adjacent thereto. Similarly it is preferred that the wells comprise a substantially parallel array of tubular walls having closed bottom ends and which tubular wells may be maintained in their spaced array by upstanding webs that interconnect each of the tubular wells to the tubular wells adjacent thereto. The webs advantageously reinforce the trays and maintain rigidity for the tubular walls (which may be particularly desirable when the trays are to be subjected to shock forces, for example, by centrifuging).

The depth of entry of the tubular spigots on the test tray into the wells of the collection tray is conveniently restricted by abutment of bottom edges of the aforementioned webs of the test tray on the top of the collection tray. The test tray may have a downwardly extending peripheral wall that extends around the chambers and the depth of entry of the tubular spigots into the wells may also, or alternatively, be restricted by abutment of a lower end of said peripheral wall with the top of the collection tray. The bottom end of the peripheral wall may be rebated to provide a skirt within which is accommodated a substantially corresponding profile on an upper surface of the collection tray so that the collection tray is received as a reasonably good fit within the skirt of the test tray.

The tray assembly of the present invention was primarily developed for chromatographic assay purposes and in this context an appropriate chromatographic medium will be carried within each chamber and retained therein from being flushed through the bottom opening with the test liquid. The chromatographic medium is retained in the chamber by an internal shoulder on a wall of the chamber, such shoulder underlying the medium. Each chamber has a circular section lower part length to be of cylindrical form and a small annular flange is provided at the bottom end of each tubular spigot to provide the shoulder for restraining the chromatographic medium as aforementioned. The chromatographic medium selected may be of a form conventional for the extraction cartridges sold under the Trade Mark BOND ELUT by Analytichem International and may be sandwiched within the chamber between underlying and overlying frits which are usually of a porous nature. Typically the frits are nylon or sintered discs which serve to retain the medium in position within the chamber and provide filtering characteristics. The lower part length of the chamber within which the chromatographic or other separation medium is accommodated has a substantially cylindrical profile so that, preferably, face-to-face contact occurs between the aforementioned frits and the chamber wall, or when a relatively rigid or non-resilient cylindrical slug of the separation medium is used, to ensure a reasonable length of face-to-face contact is provided between the medium and the chamber wall. The provision of the relatively long, cylindrical, contact faces is to alleviate difficulties which have been encountered where the frits and/or the chromatographic medium are of a relatively rigid or non-resilient material and a cylindrical slug of such a material when located in a frusto conical chamber provides only a narrow circular line of contact therewith (this contact may be inadequate to maintain the material in position and may also permit seepage of the test liquid between the material and the wall of the chamber). The constant cross section of the cylindrical part of the chamber also alleviated any need to use frits of different dimensions in different part lengths of the chamber.

### DRAWINGS

One embodiment of an assay tray assembly constructed in accordance with the present invention will now be described, by way of example only, with reference to the accompanying illustrative drawings, in which:-
Figure 1 is a plan view of the assay test tray removably mounted on a collection tray in accordance with the invention;
Figure 2 is a side view of the assembly shown in Figure 1 and shows the assembly in part section taken on the line II-II of Figure 1, and
Figure 3 is an enlarged sectional view of a chamber in the test tray of the assembly with the chamber accommodating a chromotographic medium.

### DETAILED DESCRIPTION OF DRAWINGS

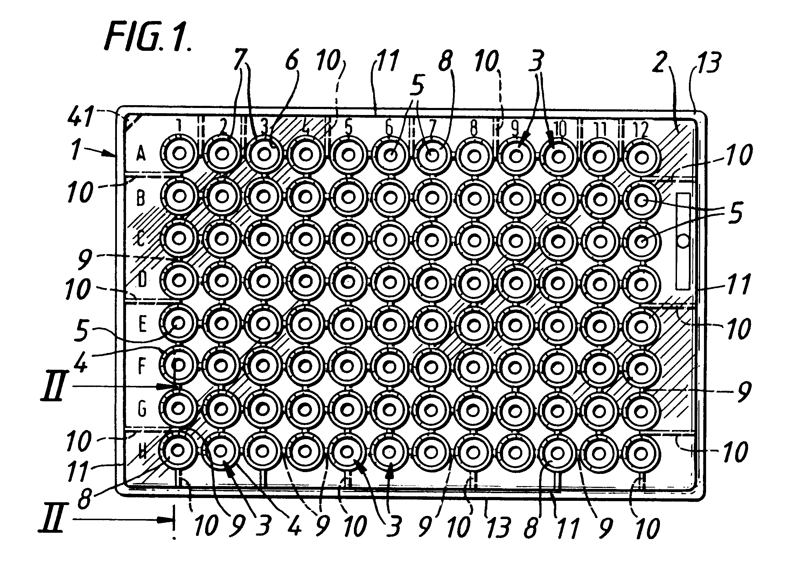

The assay tray assembly illustrated has a test tray 1 and a collection tray 72 each of which is a one piece unit injection moulded in plastics. The test tray 1 has an oblong rectangular and flat upper wall surface 2 opening into which are ninety six chambers 3 disposed in a spaced array similarly to a conventional microtiter plate. In this array there are eight rows extending parallel to a longer side edge of the face 2 with twelve chambers 3 in each row and the chambers are spaced to provide twelve parallel columns with eight chambers in each column extending perpendicularly to the rows. The rows are identified by the letters A to H shown in Figure 1 and the columns by the numerals 1 to 12 shown in Figure 1, these letters and numerals are moulded into the surface 2 so that by reference thereto each chamber may be individually identified. The chambers 3 are identical and parallel to each other and each is formed by a tubular wall 4 extending downwardly from the top opening thereof in the upper wall 2 to provide a bottom opening or outlet port 5. The lower part length 6 of each chamber 3 is cylindrical and communicates with a co-axial frusto conical upper part length 7 which tapers to converge from the top opening to the cylindrical chamber part 6. The outlet port 5 is formed by an annular flange 8 at the bottom end of the tubular wall 4 and which provides a shoulder within the chamber 3. The tubular walls 4 are maintained in their spaced array by their moulding into the upper wall 2, by upstanding webs 9 which interconnect each tubular wall 4 to the tubular walls 4 adjacent thereto and also by a spaced array of upstanding webs 10 which interconnect the webs 9 (and possibly the tubular walls 4) to a peripheral side wall 11. The side wall extends downwardly from the periphery of the upper wall 2 and surrounds the array of tubular walls 4 in the test tray. The webs 9 and 10 serve to reinforce the structure of the tray and their form will be apparent to persons skilled in the art of injection moulding.

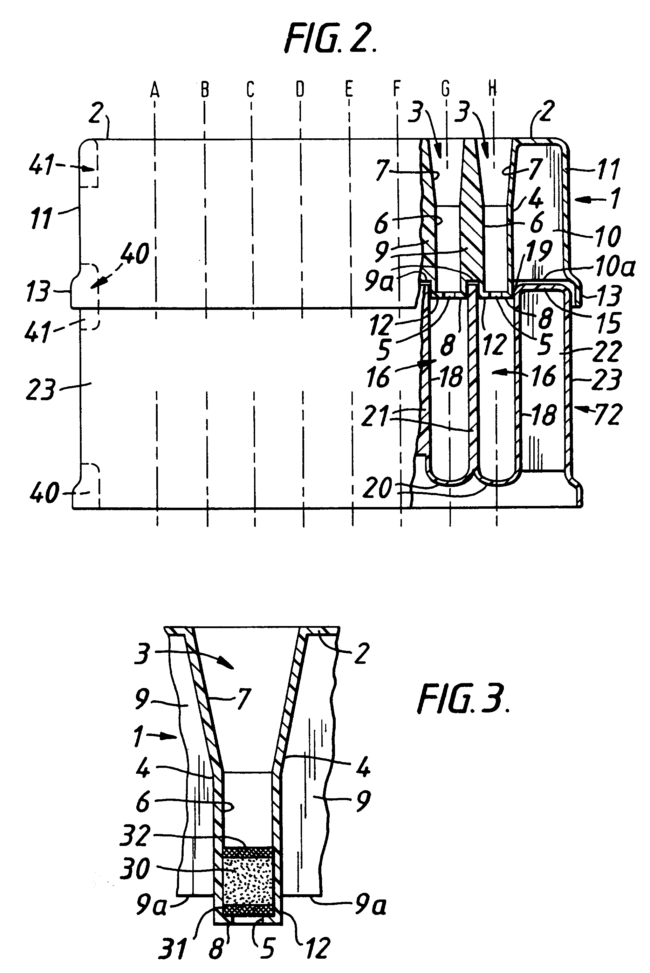

It will be seen from Figures 2 and 3 that the bottom part length of each tubular wall 4 for a chamber projects downwardly beyond the lowermost edges 9a and 10a of the webs 9 and 10 respectively to form tubular spigots 12. The bottom end of the peripheral wall 11 is formed as a peripheral skirt 13 which extends downwardly beyond the lowermost edges 9a and 10a similarly to the tubular spigots 12.

The collection tray 72 has an upper wall surface 15 opening into which is a spaced array of ninety six wells 16. The wells 16 are identical to each other and disposed in rows and columns which correspond to the rows and columns of the chambers 3 (particularly with respect to the tubular spigots 12). The upper surface 15 has (not shown) moulded-in identification letters A to H and numerals 1 to 12 in a similar manner to the test tray 1 so that each individual well 16 can readily be identified by reference to the indicia letters and numerals. The profile of the surface 15 corresponds to that of the surface 2. Each well is formed by a cylindrical tubular wall part 18 which forms a mouth 19 in the upper wall 15 from which it extends downwardly and the bottom end of the well is closed by a hemispherical wall part 20. The well walls 18 are retained in their spaced array and in parallel relationship with each other by the upper surface wall 15, upstanding webs 21 which interconnect each tubular wall 18 with the tubular walls 18 adjacent thereto, and upstanding webs 22 which interconnect the webs 21 (or possibly the walls 18) with a peripheral side wall 23 of the collection tray. The webs 21 and 22 are similar to the webs 9 and 10 and the peripheral side wall 23 extends downwardly from the upper surface wall 15 to surround the wells. For convenience in Figure 2 the sectioned part illustrates chambers 3 and wells 16 in columns G and H only - it being appreciated that chambers and wells are similarly provided in the rows indicated at A to F.

In use it is intended that the test tray 1 will be mounted in overlying relationship on the collection plate 72 with the upper wall surfaces 2 and 15 similarly orientated for the reference letters A to H and reference numerals 1 to 12 to correspond in directly overlying relationship. In such mounting the trays engage for the skirt 13 of the test tray 1 to receive the upper surface 15 of the collection tray 72 in substantially complementary manner while the tubular spigots 12 are received within the mouths 19 of the corresponding wells 16 as shown in Figure 2. The depth to which the tubular spigots 12 enter the wells 16 is determined by the abutment of the lower edges 9a and 10a of the webs in the test tray on the upper wall surface 15 of the collection tray. Alternatively, or in addition, the depth of insertion of the tubular spigots can be restricted by abutment of the peripheral edge of the upper wall surface 15 against an internal shoulder formed by the skirt 13 on the test tray.

For the purpose of conducting an assay a selective separation medium will be carried in each of the chambers 3 of the test tray. Liquid under analysis is intended to flow over or through the separation medium and pass from the outlet ports 5 to be deposited in the wells 16 with which the tubular spigots 12 respectively communicate. The tray assembly and test tray 1 illustrated are primarily intended for use in conducting a chromatographic assay and a selective chromatographic medium is located in each of the chambers 3 in a similar manner to the previously mentioned known extraction cartridges sold under the Trade Mark BOND ELUT. For convenience the chromatographic medium has been omitted from the test tray in Figures 1 and 2 but such a medium is shown at 30 in the chamber 3 of Figure 3. The chromatographic medium is in the form of a cylindrical plug 30 which is closely received within the cylindrical bore 6 of the chamber between an underlying cylindrical frit 31 and an overlying cylindrical frit 32. The frits 31 and 32 are typically nylon filters which are closely received in the cylindrical bore 6 so that the cylindrical surfaces of the chromatographic plug 30 and frits 31 and 32 make face-to-face contact with the bore 6. The plug 30 is retained in the bottom of the chamber 3 by abutment of the lower frit 31 against the internal shoulder formed by the annular flange 8. In practice it is likely that the test tray 1 will be commercially available with selective chromatographic mediums 30 and frits 31, 32 fitted so that the trays will be purchased as appropriate for an intended assay. As an alternative to a plug, the chromatographic medium can be inserted in the chambers in powder, slurry or other form.

Predetermined volumes of test liquid or liquids which are to be analysed are pipetted or otherwise deposited in each of the chambers 3 so that such liquid in its flow through the chambers 3 is subjected to the chromatographic medium 30 in that chamber and the liquid emerging from the outlet ports 5, the percolate or eluate as the case may be, is collected in the respective wells 16 to be available for analysis. The trays of the assembly are particularly intended to be suitable for handling by automatic equipment where it is likely that the test liquid or liquids will be pipetted simultaneously into the eight chambers A to H of the first column and the remaining columns of chambers are then successively charged with the test liquid. The frusto conical part 7 of the chamber 3 is desirable to provide a relatively large reservoir for the test liquid.

In conducting a chromatographic assay it will often be essential to identify the wells 16 into which the percolate or eluate (as the case may be) has been collected from the chambers 3 which respectively communicate with those wells. For this purpose the collection tray 72 will conveniently be orientated so that the identification grid of letters A to H and numerals 1 to 12 correspond between the overlying trays and the references by which a particular chamber 3 is identified are the same as those for identifying the well 16 with which that chamber communicates. To ensure that this orientation is achieved, co-operating means may be provided between the test and collection trays so that such means inter-engages when the trays are correctly orientated to be mounted one on the other (as shown in Figure 2) but will not permit such mounting when the trays are incorrectly orientated with respect to each other. The co-operating means may simply comprise a track and track follower formed at corresponding corners of the test tray and the collection tray (as indicated at 40 in Figure 2) and which track and track follower must inter-engage as the test tray is fitted to the top of the collection tray to permit the correct mounting. As an addition, or alternatively, where the trays are intended to be handled by automatic equipment, the equipment may be programmed to sense the presence or absence of a particular location on the trays from which their correct orientation relative to the equipment and to each other may be determined; for example corresponding corners of the trays may be provided with upstanding chamfers or flats 41 on their respective side walls which are intended to be sensed by automatic handling equipment to ensure that the two trays are correctly orientated with respect to each other.

As the percolate or eluate liquid resulting from a chromatographic assay is collected in the wells 16, it will be appreciated from Figure 2 that the penetration of the tubular spigots 12 into the mouths of the respective wells alleviates the possibility of cross contamination between the liquids which emerge from the respective outlet ports 5. Furthermore the volume of each well 16 below the level of the bottom end face of the tubular spigot which it receives is sufficient to accommodate all of the percolate of eluate liquid which results from the respective chambers 3. Desirably therefore the aforementioned volume of each well is greater than the volume of each chamber 3 (although it is appreciated that the volume of test liquid which is deposited in each chamber 3 will often be the same as or less than the volume of the chamber 3 above the level of the uppermost frit 32). With such an arrangement it may be ensured that all of the percolate or eluate liquid from the chambers 3 can be accommodated within the respective wells 16 clear of the tubular spigots 12 in those wells. Consequently when the test tray is removed from the collection tray for analysis of the percolate or eluate, the possibility is alleviated of cross contamination by liquid on a tubular spigot inadvertently dripping into a well other than that within which the spigot was received.

The test and collection trays may be subjected to centrifugal forces and/or gas or air pressure differentials to promote the rate of liquid flow through the separation medium.

## Claims

1. An assay tray assembly comprising a plastics moulded test tray (1) removably mounted in overlying relationship in engagement with a plastics moulded collection tray (72); the test tray (1) having a spaced array of discrete identical upstanding chambers (3) to accommodate a predetermined volume of liquid for analysis, each chamber (3) being formed with a top opening and a bottom opening (5) for the flow therethrough of the liquid, said bottom opening (5) being located in a downwardly projecting tubular spigot (12) for the respective chamber (3) and each said chamber (3) carrying a separation medium (30) to which the liquid is to be subjected during its said through-flow; the collection tray (72) having discrete upstanding wells (16) each with an upwardly opening mouth (19), said wells (16) corresponding in number to the chambers and having their mouths (19) disposed in a spaced array corresponding to that of the tubular spigots (12); the tubular spigots (12) being received one in each of said well mouths (19) for the respective wells (16) to collect liquid emanating from the chambers (3) and to alleviate cross contamination between such liquids and abutment means (9a, 10a, 15) being provided between the trays (1, 72) to restrict the depth of entry of the tubular spigots (12) into the wells (16) CHARACTERISED IN THAT the test tray (1) is formed as a one piece plastics moulding and each chamber (3) of the test tray (1) has a cylindrical lower part length (6) which communicates with the bottom opening (5), said bottom opening being formed as an outlet port (5) by an inwardly directed annular flange (8) provided in the tubular spigot part (12) of the respective chamber (3); the cylindrical lower part length (6) of each chamber (3) carrying said separation medium (30) with the medium (30) in cylindrical face-to-face contact with the cylindrical lower part length of the chamber (3) in which it is carried and said medium (30) being retained in the respective chamber (3) by abutment with the flange (8) which forms the outlet port (5) of that chamber, and wherein the volume of each well (16) below the tubular spigot (12) it receives is greater than the volume of a liquid which it is intended to receive from the chamber (3) communicating therewith.

2. An assay tray assembly as claimed in claim 1 in which the separation medium (30) is sandwiched in the lower part length (6) of its respective chamber (3) between underlying and overlying frits (31, 32) and is retained in that chamber by abutment of the underlying frit (31) with the inwardly directed flange (8).

3. An assay tray assembly as claimed in claim 2 in which the frits (31, 32) provide cylindrical face-to-face contact with the cylindrical lower part length (6) of the chamber (3) within which they are received.

4. An assay tray assembly as claimed in any one of the preceding claims in which each chamber (3) has an upper part length (7) within which is located the top opening, said upper part length (7) being frusto conical to converge as it approaches the bottom opening (5) from the top opening and being co-axial with the cylindrical lower part length (6) of the respective chamber (3).

5. An assay tray assembly as claimed in any one of the preceding claims in which the separation medium (30) is in the form of a relatively rigid or non-resilient cylindrical slug.

6. An assay tray assembly as claimed in any one of the preceding claims in which an upper surface of the test tray (1) is provided with indicia means for identifying a particular chamber (3) in the test tray (1).

7. An assay tray assembly as claimed in any one of the preceding claims in which the collection tray (72) is provided with indicia means for identifying a particular well (16) in that tray (72).

8. An assay tray assembly as claimed in claims 6 and 7 and comprising orientating means (41) which ensures a predetermined orientation is provided between the test tray (1) and the collection tray (72) when said trays are mounted in overlying and engaging relationship so that the spigot (12) of an identifiable chamber (3) will co-operate with the mouth (19) of a predetermined and identifiable well (16).

9. An assay tray for use in an assay tray assembly as claimed in any one of the preceding claims and comprising a plastics moulded test tray (1) having a spaced array of discrete identical upstanding chambers (3) to accommodate a predetermined volume of liquid for analysis, each chamber (3) being formed with a top opening and a bottom opening (5) for the flow therethrough of the liquid, said bottom opening (5) being located in a downwardly projecting tubular spigot (12) for the respective chamber (3) and each said chamber (3) carrying a separation medium (30) to which the liquid is to be subjected during its said through-flow; CHARACTERISED IN THAT the test tray (1) is formed as a one piece plastics moulding and each chamber (3) thereof has a cylindrical lower part length (6) which communicates with the bottom opening (5), said bottom opening being formed as an outlet port (5) by an inwardly directed annular flange (8) provided in the tubular spigot part (12) of the respective chamber (3); the cylindrical lower part length (6) of each chamber (3) carrying said separation medium (30) with the medium (30) in cylindrical face-to-face contact with the cylindrical lower part length of the chamber (3) in which it is carried and said medium (30) being retained in the respective chamber (3) by abutment with the flange (8) which forms the outlet port (5) of that chamber.

## Patentansprüche

1. Analysenplattenbaueinheit, umfassend eine aus Kunststoff geformte Testplatte (1), die in überlagernder Beziehung im Eingriff mit einer aus Kunststoff geformten Auffangplatte (72) abnehmbar angebracht ist; wobei die Testplatte (1) eine regelmäßige Anordnung von im Abstand angeordneten getrennten identischen aufrechten Kammern (3) zur Unterbringung eines vorbestimmten Flüssigkeitvolumens für eine Analyse aufweist, wobei jede Kammer (3) mit einer oberen Öffnung und einer unteren Öffnung (5) für das Hindurchfließen der Flüssigkeit ausgebildet ist, wobei die besagte untere Öffnung (5) in einem nach unten überstehenden röhrenförmigen Einsteckröhrchen (12) für die jeweilige Kammer (3) angeordnet ist, und jede besagte Kammer (3) ein Trennmedium (30) enthält, welchem die Flüssigkeit während ihres besagten Hindurchfließens ausgesetzt werden soll; wobei die Auffangplatte (72) getrennte aufrechte Vertiefungen (16) aufweist, jede mit einer sich nach oben öffnenden Mundöffnung (19), wobei die besagten Vertiefungen (16) in ihrer Anzahl den Kammern entsprechen und mit ihren Mundöffnungen (19) im Abstand voneinander in einer regelmäßigen Anordnung angeordnet sind, welche derjenigen der röhrenförmigen Einsteckröhrchen (12) entspricht; wobei von den röhrenförmigen Einsteckröhrchen (12) jeweils eines in jeder der besagten Vertiefungsmundöffnungen (19) für die jeweiligen Vertiefungen (16) aufgenommen wird, um Flüssigkeit aufzufangen, die aus den Kammern (3) austritt, und um eine gegenseitige Verunreinigung zwischen derartigen Flüssigkeiten zu vermindern, und wobei eine Anschlageinrichtung (9a, 10a, 15) zwischen den Platten (1, 72) vorgesehen ist, um die Eintrittstiefe der röhrenförmigen Einsteckröhrchen (12) in die Vertiefungen (16) zu begrenzen, dadurch gekennzeichnet, daß die Testplatte (1) als einstückiges Kunststofformteil ausgebildet ist, und jede Kammer (3) der Testplatte (1) ein zylindrisches unteres Teilstück (6) aufweist, welches mit der unteren Öffnung (5) in Verbindung steht, wobei die besagte untere Öffnung mittels eines im röhrenförmigen Einsteckröhrchenteil (12) der jeweiligen Kammer (3) vorgesehenen ringförmigen Flanschs (8) als Auslaßöffnung (5) ausgebildet ist; wobei das zylindrische untere Teilstück (6) jeder Kammer (3) das besagte Trennmedium (30) enthält, wobei das Medium (30) in zylindrischer Fläche-an-Fläche-Berührung mit dem zylindrischen unteren Teilstück der Kammer (3) steht, in welcher es enthalten ist, und wobei das besagte Medium (30) durch Anschlag mit dem Flansch (8), welcher die Auslaßöffnung (5) dieser Kammer bildet, in der jeweiligen Kammer (3) zurückgehalten wird, und bei welcher das Volumen jeder Vertiefung (16) unterhalb des von ihr aufgenommenen Einsteckröhrchens (12) größer als das Volumen einer Flüssigkeit ist, die sie aus der damit verbundenen Kammer (3) aufnehmen soll.

2. Analysenplattenbaueinheit nach Anspruch 1, bei welcher das Trennmedium (30) im unteren Teilstück (6) seiner jeweiligen Kammer (3) zwischen einer darunterliegenden und darüberliegenden Fritte (31, 32) eingelegt ist und durch Anschlag der darunterliegenden Fritte (31) mit dem nach innen gerichteten Flansch (8) in dieser Kammer festgehalten wird.

3. Analysenplattenbaueinheit nach Anspruch 2, bei welcher die Fritten (31, 32) für eine zylindrische Fläche-an-Fläche-Berührung mit dem zylindrischen unteren Teilstück (6) der Kammer (3) sorgen, innerhalb derer sie aufgenommen werden.

4. Analysenplattenbaueinheit nach einem beliebigen der vorangehenden Ansprüche, bei welcher jede Kammer (3) ein oberes Teilstück (7) aufweist, innerhalb dessen die obere Öffnung angeordnet ist, wobei das besagte obere Teilstück (7) kegelstumpfförmig ist, so daß es konvergiert, während es sich von der oberen Öffnung aus der unteren Öffnung (5) nähert, und zum zylindrischen unteren Teilstück (6) der jeweiligen Kammer (3) koaxial ist.

5. Analysenplattenbaueinheit nach einem beliebigen der vorangehenden Ansprüche, bei welcher das Trennmedium (30) in Form eines relativ starren oder unelastischen zylindrischen Formlings vorliegt.

6. Analysenplattenbaueinheit nach einem beliebigen der vorangehenden Ansprüche, bei welcher eine Oberseite der Testplatte (1) mit Markierungseinrichtungen zum Identifizieren einer bestimmten Kammer (3) in der Testplatte (1) versehen ist.

7. Analysenplattenbaueinheit nach einem beliebigen der vorangehenden Ansprüche, bei welcher die Auffangplatte (72) mit Markierungseinrichtungen zum Identifizieren einer bestimmten Vertiefung (16) in dieser Platte (72) versehen ist.

8. Analysenplattenbaueinheit nach Anspruch 6 und 7, und umfassend eine Ausrichteinrichtung (41), welche sicherstellt, daß für eine vorbestimmte Ausrichtung zwischen der Testplatte (1) und der Auffangplatte (72) gesorgt ist, wenn die besagten Platten in überlagernder und ineinandergreifender Beziehung zusammengesetzt sind, so daß das Einsteckröhrchen (12) einer identifizierbaren Kammer (3) mit der Mundöffnung (19) einer vorbestimmten und identifizierbaren Vertiefung (16) zusammenwirkt.

9. Analysenplatte zur Verwendung in einer Analysenplattenbaueinheit gemäß einem beliebigen der vorangehenden Ansprüche und umfassend eine aus Kunststoff geformte Testplatte (1), die eine regelmäßige Anordnung von im Abstand angeordneten getrennten identischen aufrechten Kammern (3) zur Unterbringung eines vorbestimmten Flüssigkeitvolumens für eine Analyse aufweist, wobei jede Kammer (3) mit einer oberen Öffnung und einer unteren Öffnung (5) für das Hindurchfließen der Flüssigkeit ausgebildet ist, wobei die besagte untere Öffnung (5) in einem nach unten überstehenden röhrenförmigen Einsteckröhrchen (12) für die jeweilige Kammer (3) angeordnet ist, und jede besagte Kammer (3) ein Trennmedium (30) enthält, welchem die Flüssigkeit während ihres besagten Hindurchfließens ausgesetzt werden soll; dadurch gekennzeichnet, daß die Testplatte (1) als einstückiges Kunststofformteil ausgebildet ist, und jede Kammer (3) derselben ein zylindrisches unteres Teilstück (6) aufweist, welches mit der unteren Öffnung (5) in Verbindung steht, wobei die besagte untere Öffnung mittels eines im röhrenförmigen Einsteckröhrchenteil (12) der jeweiligen Kammer (3) vorgesehenen, nach innen gerichteten ringförmigen Flanschs (8) als Auslaßöffnung (5) ausgebildet ist; wobei das zylindrische untere Teilstück (6) jeder Kammer (3) das besagte Trennmedium (30) enthält, wobei das Medium (30) in zylindrischer Fläche-an-Fläche-Berührung mit dem zylindrischen unteren Teilstück der Kammer (3) steht, in welchem es enthalten ist, und das besagte Medium (30) durch Anschlag mit dem Flansch (8), welcher die Auslaßöffnung (5) dieser Kammer bildet, in der jeweiligen Kammer (3) festgehalten wird.

## Revendications

1. Ensemble de plateaux d'analyse comprenant un plateau d'essai (1) moulé dans le plastique monté en prise séparable, en relation de superposition, avec un plateau de collecte (72) moulé dans le plastique ; le plateau d'essai (1) possédant un réseau espacé de chambres verticales identiques séparées (3) destinées à recevoir un volume prédéterminé de liquide à analyser, chaque chambre (3) étant formée d'une ouverture supérieure et d'une ouverture inférieure (5) pour l'écoulement du liquide à travers celle-ci, ladite ouverture inférieure (5) étant située dans un ergot tubulaire (12) se projetant vers le bas de la chambre respective (3) et chacune desdites chambres (3) contenant un milieu de séparation (30) auquel le liquide doit être soumis au cours dudit écoulement traversant de celui-ci ; le plateau de collecte (72) possédant des puits verticaux séparés (16) présentant chacun une embouchure verticale (19), lesdits puits (16) correspondant en nombre aux chambres et ayant leurs embouchures (19) disposées selon un réseau espacé correspondant à celui des ergots tubulaires (12) ; chacun des ergots tubulaires (12) étant reçu dans lesdites embouchures de puits (19) des puits respectifs (16) pour collecter les liquides provenant des chambres (3) et pour réduire la contamination croisée entre de tels liquides, des moyens de butée (9a, 10a, 15) étant prévus entre les plateaux (1, 72) pour restreindre la profondeur de pénétration des ergots tubulaires (12) dans les puits (16), CARACTÉRISÉ EN CE QUE le plateau d'essai (1) est formé d'une seule pièce moulée dans le plastique et en ce que chaque chambre (3) du plateau d'essai (1) a un tronçon inférieur cylindrique (6) qui communique avec l'ouverture inférieure (5), ladite ouverture inférieure constituant un orifice de sortie (5) à proximité d'une bride annulaire (8) dirigée vers l'intérieur et prévue dans la partie formant ergot tubulaire (12) de la chambre respective (3) ; le tronçon inférieur cylindrique (6) de chaque chambre (3) contenant ledit milieu de séparation (30), le milieu (30) étant en contact de vis-à-vis cylindrique avec le tronçon inférieur cylindrique de la chambre (3) dans lequel il est contenu et ledit milieu (30) étant retenu dans la chambre respective (3) par butée contre la bride (8) qui forme l'orifice de sortie (5) de cette chambre, et dans lequel le volume de chaque puits (16) au-dessous de l'ergot tubulaire (12) qu'il reçoit est supérieur au volume d'un liquide qu'il est destiné à recevoir depuis la chambre (3) communiquant avec celui-ci.

2. Ensemble de plateaux d'analyse selon la revendication 1, dans lequel le milieu de séparation (30) est pris en sandwich dans le tronçon inférieur (6) de sa chambre respective (3) entre des frittés sous-jacent et sur-jacent (31, 32) et est retenu dans cette chambre par butée du fritté sous-jacent (31) contre la bride (8) dirigée vers l'intérieur.

3. Ensemble de plateaux d'analyse selon la revendication 2, dans lequel les frittés (31, 32) offrent un contact de vis-à-vis cylindrique avec le tronçon inférieur cylindrique (6) de la chambre (3) dans laquelle ils sont reçus.

4. Ensemble de plateaux d'analyse selon l'une quelconque des revendications précédentes, dans lequel chaque chambre (3) présente un tronçon supérieur (7) dans lequel est située l'ouverture supérieure, ledit tronçon supérieur (7) étant tronconique de façon à converger à l'approche de l'ouverture inférieure (5) depuis l'ouverture supérieure et étant coaxial avec le tronçon cylindrique inférieur (6) de la chambre respective (3).

5. Ensemble de plateaux d'analyse selon l'une quelconque des revendications précédentes, dans lequel le milieu de séparation (30) se trouve sous la forme d'un lingot cylindrique relativement rigide ou non résilient.

6. Ensemble de plateaux d'analyse selon l'une quelconque des revendications précédentes, dans lequel une surface supérieure du plateau d'essai (1) est pourvue de moyens d'indexation pour identifier une chambre particulière (3) dans le plateau d'essai (1).

7. Ensemble de plateaux d'analyse selon l'une quelconque des revendications précédentes, dans lequel le plateau de collecte (72) est pourvu de moyens d'indexation pour identifier un puits particulier (16) dans ce plateau (72).

8. Ensemble de plateaux d'analyse selon les revendications 6 et 7 et comprenant des moyens d'orientation (41) qui garantissent une orientation pré-déterminée du plateau d'essai (1) par rapport au plateau de collecte (72) lorsque lesdits plateaux sont montés en relation de superposition et en prise, de sorte que l'ergot (12) d'une chambre identifiable (3) coopèrera avec l'embouchure (19) d'un puits prédéterminé et identifiable (16).

9. Plateau d'analyse utilisé dans un ensemble de plateaux d'analyse selon l'une quelconque des revendications précédentes et comprenant un plateau d'essai (1) moulé dans le plastique possédant un réseau espacé de chambres verticales identiques séparées (3) destinées à recevoir un volume prédéterminé de liquide à analyser, chaque chambre (3) comportant une ouverture supérieure et une ouverture inférieure (5) pour l'écoulement du liquide à travers celle-ci, ladite ouverture inférieure (5) étant située dans un ergot tubulaire (12) se projetant vers le bas de la chambre respective (3) et chacune desdites chambres (3) contenant un milieu de séparation (30) auquel le liquide doit être soumis au cours dudit écoulement traversant de celui-ci ; CARACTÉRISÉ EN CE QUE le plateau d'essai (1) est formé d'une seule pièce moulée dans le plastique et en ce que chaque chambre (3) de celui-ci a un tronçon inférieur cylindrique (6) qui communique avec l'ouverture inférieure (5), ladite ouverture inférieure étant constituée d'un orifice de sortie (5) au voisinage d'une bride annulaire (8) dirigée vers l'intérieur et prévue dans la partie formant ergot tubulaire (12) de la chambre respective (3) ; le tronçon inférieur cylindrique (6) de chaque chambre (3) contenant ledit milieu de séparation (30), le milieu (30) étant en contact de vis-à-vis cylindrique avec le tronçon inférieur cylindrique de la chambre (3) dans lequel il est contenu et ledit milieu (30) étant retenu dans la chambre respective (3) par butée contre la bride (8) qui forme l'orifice de sortie (5) de cette chambre.
